# FASCICULE DE BREVET EUROPEEN

(11) **EP 3 531 909 B1**
(45) Date de publication et mention de la délivrance du brevet: **22.07.2020**
(21) Numéro de dépôt: 17787455.9
(22) Date de dépôt: 27.10.2017
(51) Int. Cl.: A61B 5/0478, A61B 5/00

(54) **DISPOSITIF D'ACQUISITION DE SIGNAUX D'ACTIVITE CEREBRALE**
VORRICHTUNG ZUR ERFASSUNG VON HIRNAKTIVITÄTSSIGNALEN
DEVICE FOR ACQUIRING BRAIN ACTIVITY SIGNALS

(30) Priorité: 28.10.2016 FR 1660503
(43) Date de publication de la demande: 04.09.2019
(73) Titulaire: Urgotech, 75116 Paris (FR)
(72) Inventeur: LANTENOIS, Philippe, 69600 Oullins (FR); MAZOYER, Joseph, 69110 Ste Foy Les Lyon (FR)
(74) Mandataire: Plasseraud IP
(86) Numéro de dépôt international: PCT/EP2017/077594
(87) Numéro de publication internationale: WO 2018/078091

(56) Documents cités:
- WO-A2-2012/170816
- JP-U- H0 258 410
- KR-A- 20030 002 677
- US-A- 2 426 958
- US-A1- 2009 171 181
- US-A1- 2012 143 020
- US-B1- 7 231 723

## Description

La présente invention concerne un dispositif d'acquisition d'un encéphalogramme destiné à être positionné sur la tête d'un utilisateur de manière à acquérir un signal d'activité cérébrale, notamment pour permettre la caractérisation et/ou le traitement du sommeil, des troubles de l'hyperactivité, des troubles migraineux, des troubles de la mémoire, des troubles cognitifs, etc ... de l'utilisateur. L'invention concerne également un procédé de mise en position d'un tel dispositif d'acquisition sur la tête d'un utilisateur, actionné éventuellement par l'utilisateur lui-même.

Ainsi, pour caractériser notamment le sommeil d'un individu, il est connu d'identifier des fuseaux de sommeil (ou « *sleep spindles* » selon la dénomination Anglo-Saxonne). Les fuseaux de sommeil sont des signaux d'activité électrique cérébrale de fréquences généralement comprises entre 9 et 16 Hz (Molle et al., 2011) et d'amplitude allant de 25 à 150 microvolts. On trouve des fuseaux de sommeil de basses fréquences et de hautes fréquences, variables et spécifiques à chaque individu. Les fuseaux de sommeil durent généralement entre 0,5 à 2 secondes et sont le produit d'une activité du réseau réticulo-thalamo cortical. Il a été démontré que la production de fuseaux de sommeil ayant une forte densité est associée à un sommeil efficace.

L'identification des fuseaux de sommeil est possible par l'acquisition de signaux d'activité cérébrale au niveau de positions spécifiques de la tête de l'utilisateur, notamment au niveau des positions C3, C4 et/ou Cz définies par le « système international 10/20 » (voir notamment le document WO-A1-2009/061920).

Le système international 10/20 est notamment mentionné au travers de la demande de brevet WO-A1-2009/061920. Ce système, illustré sur la figure 1 (selon la nomenclature combinatoire modifiée ou « modified combinatorial nomenclature », Directives N°5 : Guidelines for Standard Electrode Position Nomenclature, American Clinical Neurophysiology Society, 2006), est en fin de compte une méthode internationalement reconnue de localisation du positionnement possible d'au moins une électrode à la surface d'un crâne humain dans le contexte de réalisation d'un électroencéphalogramme. Dans ce système, chaque électrode est ainsi identifiée par une lettre codant sa position par rapport aux grandes régions cérébrales et un numéro, ou la lettre z qui définit l'hémisphère (Jasper, 1958) :
- les lettres F, T, C, P, et O indicent respectivement les régions Frontale, Temporale, Centrale, Pariétale et Occipitale,
- les numéros pairs (2, 4, 6, 8) correspondent à l'hémisphère droit,
- les numéros impairs (1, 3, 5, 7) correspondent à l'hémisphère gauche,
- la lettre z indice les électrodes situées sur la ligne médiane.

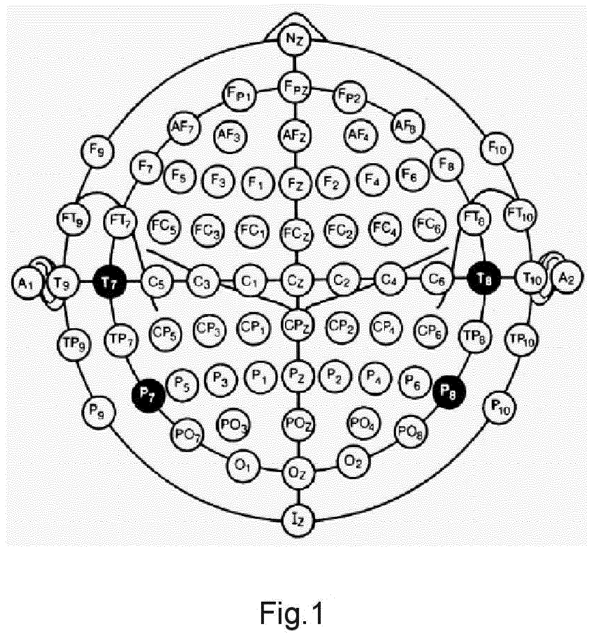

La figure 1 illustre donc les différentes positions de ce système international 10/20.

Une autre application potentielle concerne les troubles de déficit de l'attention avec ou sans hyperactivité (TDAH). Dans cette application, l'acquisition de signaux d'activité cérébrale peut se faire également au niveau des positions C3, C4, et/ou Cz (éventuellement et/ou CPz et/ou FCz) du système international 10/20.

Une autre application encore concerne, quant à elle, les troubles de la mémoire, notamment dans le cadre de la maladie d'Alzheimer. Dans cette application, l'acquisition de signaux d'activité cérébrale peut, elle, se faire notamment au niveau des positions C3, C4, T7 et/ou T8 du système international 10/20, les positions T7 et T8 correspondant respectivement aux positions T3 et T4 selon une précédente nomenclature simplifiée de ce système international 10/20. Une variante envisagée sérieusement pourrait consister en une acquisition de ces signaux d'activité cérébrale au niveau des positions C3, C4, CPz et/ou FT7 du système international 10/20.

Il est également connu de réaliser l'acquisition des signaux d'activité cérébrale en disposant sur la tête d'un utilisateur un casque muni d'une pluralité d'électrodes. A titre d'exemple, le document US-B-4,967,038 a trait à une cagoule sur laquelle sont montées des électrodes. La cagoule est réalisée en tissu extensible de sorte qu'elle peut s'adapter à différentes tailles et formes de têtes.

Toutefois, un inconvénient majeur de cette cagoule est que les électrodes sont mal positionnées contre le cuir chevelu de l'utilisateur car elles ont tendance à pivoter sur elles-mêmes, rendant l'acquisition insatisfaisante.

Un autre exemple de casque d'acquisition est décrit dans le document WO-A1-2009/045407 dans lequel le casque comprend une armature formée par des bandes inextensibles enserrant la tête de l'utilisateur. Les électrodes sont montées sur ces bandes inextensibles. Pour permettre au casque de s'adapter à différentes géométries de têtes, des bandes extensibles sont disposées entre certaines bandes inextensibles pour pouvoir éloigner ou écarter les bandes inextensibles les unes par rapport aux autres. De plus, des éléments élastiques sont disposés entre les électrodes pour plaquer les électrodes contre le cuir chevelu de l'utilisateur. Le document US-A-2,426,958 décrit un autre support d'électrodes.

Toutefois, ce casque a pour principal inconvénient de ne permettre qu'un réglage global du casque par rapport à la géométrie de la tête de l'utilisateur. En effet, les bandes extensibles apportent des degrés de liberté localisés permettant au casque de s'adapter à la tête de l'utilisateur et donc de modifier la distance entre les électrodes. Toutefois, le casque ne permet pas de connaître et de quantifier la position spécifique de chacune des électrodes. Dès lors, une utilisation ultérieure du casque sur un même utilisateur ne garantit pas que les électrodes soient placées à une position adaptée à la morphologie de l'utilisateur. L'impossibilité de disposer les électrodes dans une position prédéterminée et adaptée à l'utilisateur augmente les risques d'un mauvais positionnement des électrodes, notamment dans le cas où le sujet cherche à disposer lesdites électrodes seul, sans aide extérieure. De plus, la mise en position du casque est complexe et chronophage.

Enfin, le document WO-A1-2015/153278 traite également d'un casque d'acquisition de signaux d'activité cérébrale comprenant une pluralité de bandes support destinées à s'étendre autour de la tête de l'utilisateur pour l'enserrer et une pluralité d'électrodes montées sur les bandes support. Le casque comprend également une série de fils s'étendant entre plusieurs des bandes support et passant à travers une ou plusieurs des électrodes de manière coulissante. Des éléments d'ajustement sont reliés aux fils pour permettre de réduire ou d'augmenter la distance séparant les bandes support de manière à plaquer les électrodes contre la tête de l'utilisateur. Les bandes support et les fils peuvent être élastiques pour améliorer le plaquage des électrodes contre la tête de l'utilisateur.

Toutefois, ce casque présente les mêmes inconvénients que le casque décrit ci-avant car l'adaptation à la morphologie de la tête de l'utilisateur est réalisée de manière globale, sans laisser la possibilité d'un réglage spécifique et quantifiable de la position des électrodes.

Il existe donc un besoin pour un dispositif d'acquisition de signaux de l'activité cérébrale dont le positionnement des électrodes sur la tête d'un utilisateur est amélioré, notamment en permettant de faciliter le positionnement des électrodes au niveau d'une position prédéterminée sur la tête d'utilisateurs de morphologies différentes.

À cette fin, la présente invention propose un dispositif d'acquisition destiné à être positionné sur la tête d'un utilisateur pour réaliser un encéphalogramme, la tête de l'utilisateur étant définie selon un plan transversal, un plan sagittal et un plan frontal, selon la revendication 1.

Selon un mode de réalisation du dispositif, celui-ci comprend en outre :
- un support postérieur ;
- un support antérieur apte à coopérer avec le support postérieur pour former une portion annulaire destinée à enserrer la tête de l'utilisateur suivant le plan transversal et/ou apte à coopérer avec le support postérieur pour former une portion en arceau destinée à enserrer la tête de l'utilisateur suivant le plan sagittal,
- un support supérieur sur lequel est montée l'électrode d'acquisition, le support supérieur étant monté sur le support antérieur ou postérieur.

Selon un autre mode de réalisation du dispositif, celui-ci comprend en outre un troisième moyen d'ajustement de la position de l'électrode d'acquisition suivant un troisième plan choisi parmi les plans transversal, sagittal et frontal de la tête de l'utilisateur, les premier, deuxième et troisième plans étant différents, les premier, deuxième et troisième moyens d'ajustement de la position de l'électrode d'acquisition étant de préférence actionnables indépendamment les uns des autres.

Selon un autre mode de réalisation du dispositif, le support supérieur est associé au support postérieur, dans lequel :
- le premier moyen d'ajustement est apte à déplacer l'électrode d'acquisition par rapport au support supérieur selon une première direction comprise dans le plan frontal ;
- le deuxième moyen d'ajustement est apte à déplacer le support antérieur par rapport au support postérieur selon une deuxième direction comprise dans le plan sagittal; et
- le troisième moyen d'ajustement est apte à déplacer le support antérieur par rapport au support postérieur selon une troisième direction comprise dans le plan transversal

Selon un autre mode de réalisation du dispositif, le premier moyen d'ajustement comprend un curseur apte à coulisser selon la première direction comprise dans le plan frontal dans un logement frontal formé dans le support supérieur, l'au moins une électrode d'acquisition étant montée sur le curseur.

Selon un autre mode de réalisation du dispositif, les deuxième et troisième moyens d'ajustement comprennent :
- une tige formée sur le support antérieur ;
- un logement formé dans le support postérieur et dans lequel la tige est apte à coulisser ; et
- un moyen de réglage direct ou indirect de la position de la tige montée sur le support postérieur et apte à coopérer avec la tige pour faire coulisser la tige à l'intérieur du logement de manière à déplacer le support antérieur par rapport au support postérieur.

Selon un autre mode de réalisation du dispositif, les deuxième et troisième moyens d'ajustement comportent au moins une saillie et au moins un creux formés sur la tige ou le moyen de réglage direct ou indirect, respectivement, la saillie et le creux étant aptes à coopérer ensemble pour maintenir la tige dans une position prédéterminée par rapport au logement.

Selon un autre mode de réalisation du dispositif, le dispositif comprend une première, et/ou une deuxième et/ou une troisième électrodes d'acquisition configurées pour être positionnées au contact de la tête de l'utilisateur au niveau de l'une des positions C3, C4 et/ou Cz du système international 10/20, respectivement, et dans lequel les première, et/ou deuxième et/ou troisième électrodes d'acquisition sont montées sur le support supérieur.

Le dispositif peut comprendre en outre une électrode d'acquisition configurée pour être positionnée au contact de la tête de l'utilisateur au niveau de la position CPz du système international 10/20, et/ou une électrode d'acquisition configurée pour être positionnées au contact de la tête de l'utilisateur au niveau de la position FT7 du système international 10/20.

Alternativement, le dispositif peut comprendre en outre une électrode d'acquisition configurée pour être positionnée au contact de la tête de l'utilisateur au niveau de la position T7 du système international 10/20, et/ou une électrode d'acquisition configurée pour être positionnées au contact de la tête de l'utilisateur au niveau de la position T8 du système international 10/20.

Selon un autre mode de réalisation du dispositif, le support antérieur comprend une portion d'appui configurée pour prendre appui sur une oreille de l'utilisateur lorsque le dispositif d'acquisition est disposé sur la tête de l'utilisateur.

Selon un autre mode de réalisation du dispositif, la portion d'appui comprend au moins une électrode de référence configurée pour être positionnée au contact de la tête de l'utilisateur, de préférence en regard de l'un des processus mastoïde de l'utilisateur.

Selon un autre mode de réalisation du dispositif, le support supérieur est monté sur le support postérieur, le support postérieur comprenant un évidement apte à indiquer une position de référence du support postérieur, l'évidement étant de préférence configuré pour être disposé en regard de la protubérance occipitale externe, de préférence au niveau de l'inion, de la tête de l'utilisateur.

Selon un autre mode de réalisation, le dispositif comprend en outre :
- une unité de traitement connectée à l'au moins une électrode d'acquisition et, le cas échéant, à l'au moins une électrode de référence pour traiter des informations transmises par l'au moins une électrode d'acquisition et l'au moins une électrode de référence ;
- une unité de transmission des informations traitées par l'unité de traitement.

De plus, l'invention concerne également un procédé de mise en position d'un dispositif d'acquisition sur la tête d'un utilisateur pour réaliser un encéphalogramme, la tête de l'utilisateur étant définie selon un plan transversal, un plan sagittal et un plan frontal, le procédé comprenant les étapes consistant à :
- fournir un dispositif d'acquisition tel que décrit ci-dessus ;
- disposer le dispositif d'acquisition sur la tête de l'utilisateur ;
- actionner le troisième moyen d'ajustement pour enserrer la tête de l'utilisateur,
- actionner le deuxième moyen d'ajustement de manière à placer le support supérieur équidistant d'une extrémité distale de la portion antérieure transversale et d'une d'extrémité distale de la portion postérieure transversale,
- actionner le premier moyen d'ajustement pour ajuster la position de l'au moins une électrode d'acquisition suivant le plan frontal;
dans lequel les étapes d'actionnement des premier et deuxième moyens d'ajustement sont réalisées de manière indépendante.

Selon un mode de réalisation du procédé, celui-ci comprend en outre les étapes consistant à :
- déterminer une première et une deuxième positions d'ajustement des premier et deuxième moyens d'ajustement, respectivement ;
- mettre en mémoire les première et deuxième positions d'ajustement ;
- actionner les premier et deuxième moyens d'ajustement de manière à les positionner dans des positions d'ajustement différentes des première et deuxième positions d'ajustement ;
- enlever le dispositif d'acquisition de la tête de l'utilisateur ;
- disposer à nouveau le dispositif d'acquisition sur la tête de l'utilisateur ;
- actionner les premier et deuxième moyens d'ajustement de manière à les disposer selon les première et deuxième positions d'ajustement.

Selon un autre mode de réalisation du procédé, le dispositif d'acquisition est un dispositif d'acquisition tel que décrit ci-dessus, dans lequel l'étape consistant à disposer le dispositif d'acquisition sur la tête de l'utilisateur comprend les étapes consistant à :
- déplacer le support antérieur par rapport au support postérieur de manière à ce que la portion annulaire ait une circonférence égale ou supérieure à la circonférence maximale de la tête de l'utilisateur prise dans le plan transversal ;
- positionner le dispositif d'acquisition sur la tête de l'utilisateur de manière à ce que la portion d'appui prenne appui sur une oreille de l'utilisateur ;
- positionner le dispositif d'acquisition sur la tête de l'utilisateur de manière à ce que l'évidement du support postérieur soit en regard de la protubérance occipitale externe de la tête de l'utilisateur.

D'autres caractéristiques et avantages de l'invention apparaîtront à la lecture de la description qui suit d'un mode de réalisation préféré de l'invention, donnée à titre d'exemple et en référence aux dessins annexés.

La figure 1 déjà mentionnée plus haut représente schématiquement les positions dans le système international 10/20. Les figures 2 à 4 représentent schématiquement plusieurs vues en perspective d'un exemple de dispositif d'acquisition de signaux d'activité cérébrale disposé sur la tête d'un utilisateur, dans le cadre de l'étude des troubles du sommeil de cet utilisateur.

La figure 5 représente un diagramme illustrant un procédé de caractérisation du sommeil mise en œuvre par le dispositif d'acquisition des figures 2 à 4.

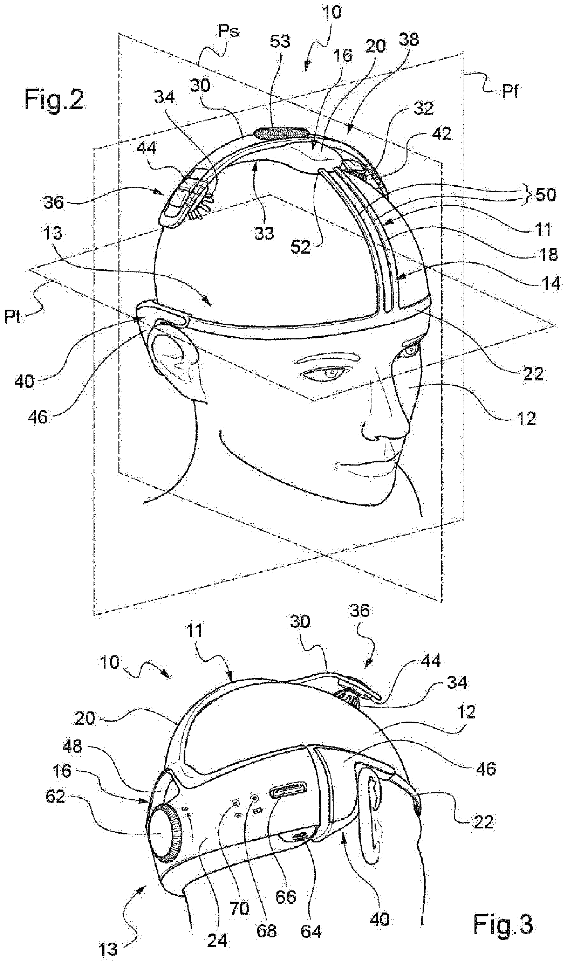

En référence à la figure 2, il est proposé un dispositif d'acquisition 10 destiné à être disposé sur une tête d'un utilisateur 12 pour acquérir des signaux d'activité cérébrale représentatifs des ondes cérébrales de l'utilisateur 12. Ces signaux d'activité cérébrale permettent notamment de réaliser un encéphalogramme. La tête de l'utilisateur 12 est définie selon un plan transversal Pt, un plan sagittal Ps et un plan frontal Pf, formant un repère orthonormé. De manière alternative, il est possible également de définir la tête de l'utilisateur 12 par toute combinaison de plans ou tout repère.

Le dispositif d'acquisition 10 est destiné à acquérir des signaux d'activité cérébrale permettant entre autres, dans un second temps, d'identifier des fuseaux de sommeil, ou d'autres ondes souhaitées. Pour cela, le dispositif d'acquisition 10 permet d'acquérir l'ensemble des signaux d'activité cérébrale pouvant être captés en regard de la disposition de l'une au moins des électrodes en C3, C4 ou Cz, de manière à arriver à ne récupérer, par l'intermédiaire de filtres, que les signaux ayant par exemple une fréquence comprise entre 9 et 16 Hz, une amplitude supérieure à 15 µV et une durée comprise entre 0.5 s et 2 s. Il est bien entendu possible de la même manière de récupérer d'autres signaux ayant des fréquences, des amplitudes et des durées autres que celles décrites *supra,* notamment afin de caractériser d'éventuels troubles de l'hyperactivité ou migraineux chez un utilisateur. Le dispositif d'acquisition 10 peut donc être utilisé comme un dispositif ou système de caractérisation du sommeil tel que décrit dans la demande de brevet FR 1556421 (de la société Urgotech) ou dans un procédé de caractérisation du sommeil également proposé ci-après dans ce document.

Le dispositif d'acquisition 10 est de la forme d'un casque apte à reposer et à se maintenir sur la tête de l'utilisateur 12. Le dispositif d'acquisition 10 s'étend au-dessus de la tête de l'utilisateur 12 suivant le plan sagittal Ps sous la forme d'une portion en arceau 11 et autour de la tête de l'utilisateur 12 sous la forme d'une portion annulaire 13, ou couronne, pour enserrer la tête de l'utilisateur 12. La portion en arceau 11 est reliée au niveau de ses extrémités à la portion annulaire 13 pour pouvoir faire reposer le dispositif d'acquisition 10 sur la tête de l'utilisateur 12.

De manière alternative à la portion annulaire 13 et à la portion en arceau 11, toute autre géométrie permettant le maintien du dispositif d'acquisition 10 sur la tête de l'utilisateur 12 peut être utilisée. En particulier, le dispositif d'acquisition 10 peut comporter une ou plusieurs portions en arceau 11 s'étendant suivant un plan parallèle ou décalé angulairement par rapport au plan sagittal Ps. De plus, la portion annulaire 13 peut être discontinue ou entourer partiellement la tête de l'utilisateur 12 tout en permettant le maintien du dispositif d'acquisition 10 sur la tête de l'utilisateur 12. De manière similaire, la portion en arceau 11 peut être discontinue ou parcourir partiellement la tête de l'utilisateur 12.

Pour permettre l'acquisition des signaux d'activité cérébrale, le dispositif d'acquisition 10 peut comprendre l'une au moins d'une première électrode d'acquisition 32, d'une deuxième électrode d'acquisition 34 ou d'une troisième électrode 33 d'acquisition destinées à être positionnées au niveau des positions C3, C4 et Cz, respectivement, du système international 10/20. Dans ces positions C3, C4 et Cz, les première 32, deuxième 34 et troisième 33 électrodes d'acquisition sont aptes, prises seules ou en combinaison, à acquérir des signaux d'activité cérébrale permettant notamment d'identifier des fuseaux de sommeil. Pour faciliter le positionnement des première 32, deuxième 34 et troisième 33 électrodes d'acquisition, ces dernières sont montées sur la portion en arceau 11 parcourant le sommet de la tête de l'utilisateur 12. De manière alternative, le dispositif d'acquisition 10 peut n'être pourvu que d'une seule électrode d'acquisition choisie parmi les première 32, deuxième 34 et troisième 33 électrodes d'acquisition.

De plus, le dispositif d'acquisition 10 peut comporter une électrode de référence permettant de réaliser une mesure de référence par rapport aux signaux d'activité cérébrale acquis avec les première 32, deuxième 34 et/ou troisième 33 électrodes d'acquisition. Cette électrode de référence est de préférence positionnée en regard de l'un des processus mastoïde de l'utilisateur 12. De plus, le dispositif d'acquisition 10 peut comporter une électrode passive (non représentée) permettant de réaliser la masse du dispositif d'acquisition 10. Cette électrode passive est de préférence disposée également en regard de l'un des processus mastoïde de l'utilisateur 12, par exemple en regard de l'autre processus mastoïde que celui où est disposée l'électrode de référence. De manière alternative, les première 32, deuxième 34 et/ou troisième électrodes 33 d'acquisition peuvent réunir en outre une fonction de référence et/ou de masse pour permettre au dispositif d'acquisition 10 de s'affranchir de l'électrode de référence et/ou de l'électrode de masse.

Les première 32, deuxième 34 et troisième 33 électrodes d'acquisition, l'électrode passive et l'électrode de référence peuvent être des électrodes sèches, humides, semi-sèches ou encore des électrodes semi-humides. Chaque type d'électrode peut avoir une nature particulière et différente.

Pour assurer le bon positionnement des première 32, deuxième 34 et troisième 33 électrodes d'acquisition au niveau des positions C3, C4 et Cz pour des utilisateurs 12 ayant des morphologies de tête différentes, le dispositif d'acquisition 10 comprend un premier (36, 38, 40) et un deuxième (36, 38, 40) moyens d'ajustement de la position des première 32, deuxième 34 et troisième 33 électrodes d'acquisition. Ainsi, en permettant un positionnement ajustable des première 32, deuxième 34 et troisième 33 électrodes d'acquisition, le dispositif d'acquisition 10 assure une bonne acquisition des signaux d'activité cérébrale pour des utilisateurs 12 ayant une morphologie de tête différente. C'est par exemple le cas lorsque les utilisateurs ont des âges différents.

Pour permettre un positionnement bi-axial des première 32, deuxième 34 et troisième 33 électrodes d'acquisition, les premier (36, 38, 40) et deuxième (36, 38, 40) moyens d'ajustement permettent l'ajustement des première 32, deuxième 34 et troisième 33 électrodes d'acquisition suivant un premier et un deuxième plans différents choisis parmi au moins les plans transversal Pt et sagittal Ps de l'utilisateur 12. Ainsi, les avantages associés au positionnement des première 32, deuxième 34 et troisième 33 électrodes d'acquisition sont améliorés car le dispositif d'acquisition 10 peut assurer une bonne acquisition des signaux d'activité cérébrale pour des utilisateurs 12 ayant des morphologies davantage différentes.

De plus, pour simplifier la mise en position des première 32, deuxième 34 et troisième 33 électrodes d'acquisition, les premier (36, 38, 40) et deuxième (36, 38, 40) moyens d'ajustement sont actionnables indépendamment l'un de l'autre. L'actionnement indépendant des premier (36, 38, 40) et deuxième (36, 38, 40) moyens d'ajustement permet de décomposer le mouvement des première 32, deuxième 34 et troisième 33 électrodes d'acquisition associé aux premier (36, 38, 40) et deuxième (36, 38, 40) moyens d'ajustement. Ainsi, combiné au fait que les premier (36, 38, 40) et deuxième (36, 38, 40) moyens d'ajustement permettent un ajustement dans des plans différents, une décomposition du déplacement des première 32, deuxième 34 et troisième 33 électrodes d'acquisition est possible suivant deux des plans parmi les plans frontal Pf, transversal Pt et sagittal Ps. En d'autres termes, il possible de déplacer sélectivement les première 32, deuxième 34 et troisième 33 électrodes d'acquisition suivant l'un parmi les plans transversal Pt, sagittal Ps et frontal Pf. De plus, cette décomposition permet d'obtenir une mise en position ciblée et plus précise des première 32, deuxième 34 et troisième 33 électrodes d'acquisition que dans un dispositif d'acquisition dans lequel les déplacements des électrodes d'acquisition sont combinés, comme par exemple dans le document WO-A1-2015/153278. De plus, la décomposition du positionnement des première 32, deuxième 34 et troisième 33 électrodes d'acquisition permet une quantification de la position des première 32, deuxième 34 et troisième 33 électrodes d'acquisition suivant deux des plans frontal Pf, transversal Pt et sagittal Ps permettant ainsi une répétabilité simple et rapide du positionnement des première 32, deuxième 34 et troisième 33 électrodes d'acquisition.

Pour obtenir un bon compromis entre la simplicité du dispositif d'acquisition 10 et le bon positionnement des première 32, deuxième 34 et troisième 33 électrodes d'acquisition, le premier plan suivant lequel le premier moyen d'ajustement 36 est apte à ajuster la position des première 32 et deuxième 34 électrodes d'acquisition est le plan frontal Pf. Le deuxième plan suivant lequel le premier moyen d'ajustement 36 est apte à ajuster la position des première 32 et deuxième 34 électrodes d'acquisition est choisi parmi les plans transversal Pt et sagittal Ps. De cette façon, le dispositif d'acquisition 10 est capable de positionner, notamment avec l'aide du deuxième moyen d'ajustement 38, les première 32 et deuxième 34 électrodes d'acquisition de manière à les aligner dans le plan frontal Pf. Le premier moyen d'ajustement 36 permet ensuite l'ajustement de la position des première 32 et deuxième 34 électrodes d'acquisition suivant le plan frontal Pf. Dans ce cas, le dispositif d'acquisition 10 peut être utilisé de sorte que le dispositif d'acquisition 10 est disposé sur la tête de l'utilisateur 12. Le deuxième moyen d'ajustement 38 est actionné pour enserrer la tête de l'utilisateur 12. Ensuite, le premier moyen d'ajustement 36 est actionné pour ajuster la position des première 32 et deuxième 34 électrodes d'acquisition suivant le plan frontal Pf.

Pour permettre au dispositif d'acquisition 10 de s'adapter à une gamme de morphologies et d'âge davantage différents, le dispositif d'acquisition 10 peut comporter un troisième moyen d'ajustement 40 de la position des première 32 et deuxième 34 électrodes d'acquisition suivant un troisième plan différent des premier et deuxième plans. Dans ce cas, la position des première 32 et deuxième 34 électrodes d'acquisition peut être ajustée suivant chacun des plans frontal Pf, transversal Pt et sagittal Ps. De plus, les premier 36, deuxième 38 et troisième 40 moyens d'ajustement sont actionnables de manière indépendante pour permettre une décomposition des mouvements des première 32 et deuxième 34 électrodes d'acquisition et ainsi simplifier leur mise en position. L'ajout d'un troisième moyen d'ajustement 40 permet d'obtenir les avantages associés aux premier et deuxième moyens d'ajustement mentionnés ci-dessus de manière améliorée. A titre d'exemple, le dispositif d'acquisition 10 présenté sur les figures 2 à 4 et comprenant les premier 36, deuxième 38 et troisième 40 moyens d'ajustement est capable de s'adapter à des morphologies allant d'un utilisateur d'environ 6 ans à un utilisateur adulte.

Pour faciliter la répétabilité du positionnement des première 32, deuxième 34 et troisième 33 électrodes d'acquisition, le dispositif d'acquisition 10 est de la forme d'un casque rigide. On entend par rigide le fait que le casque est configuré de sorte que la position des première 32, deuxième 34 et troisième 33 électrodes d'acquisition par rapport aux portions annulaire 13 et en arceau 11 ne peut être modifiée que par l'actionnement de l'un ou plusieurs des premier 36, deuxième 38 et troisième 40 moyens d'ajustement, que le casque soit ou non sur la tête de l'utilisateur 12. Le positionnement des première 32, deuxième 34 et troisième 33 électrodes d'acquisition n'est donc pas conditionné au port du casque par l'utilisateur 12. La rigidité du casque permet ainsi de conserver, partiellement ou totalement, le positionnement des première 32, deuxième 34 et troisième 33 électrodes d'acquisition suivant les plans sagittal Ps, transversal Pt et frontal Pf même quand le casque n'est pas disposé sur la tête de l'utilisateur 12. A titre de comparaison, le casque décrit dans le document WO-A1-2015/153278 ne peut être considéré comme un casque rigide au sens de la présente description car, dans ce document, le retrait du casque par l'utilisateur dérègle forcément la position des électrodes par ce que la position des électrodes est conditionnée par le port du casque par l'utilisateur.

Dans le mode de réalisation décrit sur les figures 2 à 4, le dispositif d'acquisition 10 comprend un support antérieur 14 et un support postérieur 16 reliées ensemble de manière à former la portion annulaire 13 et la portion en arceau 11 décrites ci-dessus. En particulier, le support antérieur 14 comprend une portion antérieure sagittale 18 s'étendant dans le plan sagittal Ps et rejoignant une portion postérieure sagittale 20 du support postérieur 16 pour former la portion en arceau 11. De manière similaire, le support antérieur 14 comprend une portion antérieure transversale 22 reliée à une portion postérieure transversale 24 (voir figure 3) du support postérieur 16 pour former la portion annulaire 13.

Le dispositif d'acquisition 10 comprend également un support supérieur 30 associé à la portion en arceau 11. Le support supérieur 30 s'étend dans le plan frontal Pf de part et d'autre de la portion en arceau 11. En d'autres termes, le support supérieur 30 est associé à la portion en arceau 11 de manière à être disposé au sommet de la tête de l'utilisateur 12. En particulier, le support supérieur 30 est associé au support postérieur 16, au niveau de la portion postérieure sagittale 20. Lorsque le dispositif d'acquisition 10 comporte la troisième électrode 33 d'acquisition destinée à être positionnée au niveau de la position Cz, cette troisième électrode d'acquisition est sous le support supérieur 30 de manière à être en regard de la tête de l'utilisateur 12.

Le mode de réalisation du dispositif d'acquisition 10 présenté sur les figures 2 à 4 a une géométrie simple suivant les trois plans frontal Pf, transversal Pt et sagittal Ps de la tête de l'utilisateur 12. En effet, le dispositif d'acquisition 10 est formé par une portion annulaire 13 s'étendant suivant le plan transversal Pt, par une portion en arceau 11 s'étendant suivant le plan sagittal Ps et par un support supérieur 30 s'étendant suivant le plan frontal Pf. Dès lors, la configuration du dispositif d'acquisition 10 facilite l'intégration de moyens d'ajustement de la position des première 32, deuxième 34 et troisième 33 électrodes d'acquisition. En effet, grâce à cette géométrie suivant les trois plans frontal Pf, transversal Pt et sagittal Ps, il est possible de faire varier la position des première 32, deuxième 34 et troisième 33 électrodes d'acquisition en faisant varier les dimensions ou en déplaçant le support supérieur 30 suivant une direction comprise dans le plan frontal Pf, la portion en arceau 11 suivant une direction comprise dans le plan sagittal Ps et la portion annulaire 13 suivant une direction comprise dans le plan transversal Pt.

Le dispositif d'acquisition 10 comprend également un premier 42 et un deuxième 44 curseurs constituant tout ou partie le premier moyen d'ajustement 36 des première 32, deuxième 34 et troisième 33 électrodes d'acquisition. Les premier 42 et deuxième 44 curseurs sont montés dans un logement frontal sur le support supérieur 30 de part et d'autre de la portion en arceau 11. Les première 32, deuxième 34 et troisième 33 électrodes d'acquisition sont montées sur les premier 42 et deuxième 44 curseurs de manière à être positionnées au contact de la tête de l'utilisateur 12 au niveau des positions C3, C4 et Cz, respectivement. De plus, les premier 42 et deuxième 44 curseurs sont déplaçables par rapport au support 30 selon une direction comprise dans le plan frontal Pf de manière à pouvoir ajuster la position des première 32, deuxième 34 et troisième 33 électrodes d'acquisition selon le plan frontal Pf. Le déplacement des premier 42 et deuxième 44 curseurs peut être effectué manuellement. Pour permettre un positionnement plus précis et quantifiable, les premier 42 et deuxième 44 curseurs peuvent comporter des saillies aptes à coopérer avec des creux formés dans le support 30. La coopération entre les saillies et les creux permet de placer les premier 42 et deuxième 44 curseurs, et donc les première 32, deuxième 34 et troisième 33 électrodes d'acquisition, dans des positions prédéterminées. De manière alternative, les saillies peuvent être pourvues sur le support supérieur 30 et les creux sur les premier 42 et deuxième 44 curseurs. De manière encore alternative, il est possible d'adapter tout système permettant aux première 32, deuxième 34 et troisième 33 électrodes d'acquisition de prendre des positions prédéterminées.

Pour ajuster la position des première 32, deuxième 34 et troisième 33 électrodes d'acquisition suivant le plan sagittal Ps, les portions antérieure sagittale 18 et postérieure sagittale 20 sont configurées pour être déplacées l'une par rapport à l'autre, suivant une direction comprise dans le plan sagittal Ps. Le déplacement des portions antérieure sagittale 18 et postérieure sagittale 20 l'une par rapport à l'autre constitue le deuxième moyen d'ajustement 38. En parallèle du positionnement des première 32, deuxième 34 et troisième 33 électrodes d'acquisition suivant le plan sagittal Ps, le deuxième moyen d'ajustement 38 permet également d'adapter le dispositif d'acquisition 10 à la tête de l'utilisateur 12 suivant le plan sagittal Ps. En particulier, le deuxième moyen d'ajustement 38 comprend une tige sagittale 50 et un logement sagittal 52 formé dans le support postérieur 16, à l'intérieur de la portion postérieure sagittale 20. La tige sagittale 50 est apte à coulisser à l'intérieur du logement sagittal 52 pour déplacer les portions antérieure sagittale 18 et postérieure sagittale 20 l'une par rapport à l'autre. La tige sagittale 50 peut être formée par deux éléments de tige. Dans le mode de réalisation des figures 2 à 4, la tige sagittale 50 est confondue avec la portion antérieure sagittale 18. De manière alternative, la tige sagittale 50 peut être formée en saillie de la portion antérieure sagittale 18. De manière alternative à la tige sagittale 50 et au logement sagittale 52, le deuxième moyen d'ajustement 38 peut être formé par tout système permettant de déplacer les portions antérieure sagittale 18 et postérieure sagittale 20 l'une par rapport à l'autre.

Pour permettre le réglage de la position des portions antérieure sagittale 18 et postérieure sagittale 20 l'une par rapport à l'autre, le deuxième moyen d'ajustement 38 comprend également un moyen de réglage sagittal 53 de la position de la tige sagittale 50. Le moyen de réglage sagittal 53 est apte à coopérer avec la tige sagittale 50 pour faire coulisser la tige sagittale 50 à l'intérieur du logement sagittal 52 de manière à déplacer le support antérieur 14 par rapport au support postérieur 16. Le moyen de réglage sagittal 53 prend la forme d'un bouton déplaçable en rotation autour de lui-même pour faire coulisser la tige sagittale 50 à l'intérieur du logement sagittal 52. Le bouton rotatif permet une prise en main facile du deuxième moyen d'ajustement 38. De manière alternative à un bouton rotatif, le moyen de réglage sagittal 53 peut être de toute forme permettant de faire coulisser la tige sagittale 50 à l'intérieur du logement sagittal 52. Le moyen de réglage 53 est qualifié ici de moyen, de réglage direct.

Pour maintenir la tige sagittale 50 dans une position prédéterminée par rapport au logement sagittal 52, le deuxième moyen d'ajustement 38 peut comprendre au moins une saillie formée sur la tige sagittale 52 et au moins un creux formé sur le moyen de réglage sagittal 53. La saillie et le creux sont aptes à coopérer ensemble pour maintenir la tige sagittale 52 dans une position prédéterminée par rapport au logement sagittal 52. De manière alternative, la saillie peut être formée sur le moyen de réglage sagittal 53 et le creux formé sur la tige sagittale 52.

Dans une alternative tout à fait envisageable de ce mode de réalisation, le deuxième moyen d'ajustement 38 peut être disposé au niveau de la portion antérieure transversale 22 et/ou au niveau de la portion postérieure transversale 24, au moins l'une des deux portions transversales 22, 24 étant reliée au support supérieur 30 par le biais d'une portion frontale. Le moyen d'ajustement 38 se trouve alors à la jonction de portion transversale 22, 24 reliée au support supérieur 30, et de la portion frontale, de sorte à rendre mobile le support supérieur 30 dans le plan sagittal.

Le moyen d'ajustement 38 peut comprendre dans ce mode de réalisation également un moyen de réglage qui peut être un bouton déplaçable en rotation autour de lui-même, ménageant un degré de liberté au niveau de la jonction de la portion frontale et de la portion transversale 22, 24 reliée au support supérieur 30, afin de rendre mobile dans le plan sagittal le support supérieur 30 porteur d'au moins une électrode.

Selon une variante de cette alternative, le moyen de réglage peut définir des positions de réglages préétablies du support supérieur 30 dans le plan sagittal de l'individu. Eventuellement, selon ce mode de réalisation alternatif, les supports antérieur 14 et postérieur 16 peuvent ne plus être obligatoirement présents.

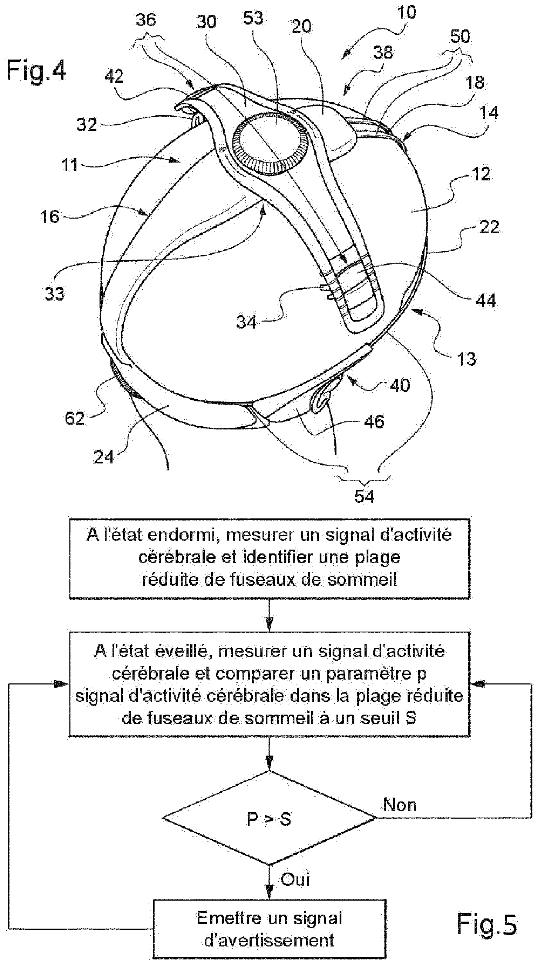

De plus, le dispositif d'acquisition 10 comprend un troisième moyen d'ajustement 40 pour permettre un ajustement de la position des première 32, deuxième 34 et troisième 33 électrodes d'acquisition suivant le plan transversal Pt. Le troisième moyen d'ajustement 40 permet aux portions antérieure transversale 22 et postérieure transversale 24 d'être déplacées l'une par rapport à l'autre suivant une direction comprise dans le plan transversal Pt. En parallèle du positionnement des première 32, deuxième 34 et troisième 33 électrodes d'acquisition suivant le plan transversal Pt, le troisième moyen d'ajustement 40 permet également d'adapter le dispositif d'acquisition 10 à la tête de l'utilisateur 12 suivant le plan transversal Pt, i.e. à la circonférence de la tête de l'utilisateur 12. En référence à la figure 4, le troisième moyen d'ajustement 40 comprend une première tige transversale 54 et une deuxième tige transversale (non représentée) formées par le support antérieur 14 de part et d'autre de la tête de l'utilisateur 12. Le troisième moyen d'ajustement 40 comprend également un premier et un deuxième logements transversaux (non représentés) formés dans le support postérieur 16 et dans lequel les première 54 et deuxième tiges transversales sont aptes à coulisser, respectivement. Le dispositif d'acquisition 10 est formé de sorte que la première tige transversale 54 et le premier logement transversal sont formés au niveau d'un premier côté du dispositif d'acquisition 10, ici le côté droit de la tête de l'utilisateur 12, et que la deuxième tige transversale et le deuxième logement transversal sont formés d'un deuxième côté du dispositif d'acquisition 10 opposé au premier côté, ici le côté gauche de la tête de l'utilisateur 12.

De manière similaire au deuxième moyen d'ajustement 38, le troisième moyen d'ajustement 40 peut comprendre un moyen de réglage transversal 62 de la position des première 54 et deuxième tiges transversales pour permettre le réglage de la position des portions antérieure transversale 22 et postérieure transversale 24 l'une par rapport à l'autre. Le moyen de réglage transversal 62 est alors apte à coopérer avec les première 54 et deuxième tiges transversales pour les faire coulisser à l'intérieur des premier et deuxième logements transversaux, respectivement, de manière à déplacer le support antérieur 14 par rapport au support postérieur 16. Le moyen de réglage transversal 62 prend la forme d'un bouton déplaçable en rotation autour de lui-même pour faire coulisser les première 54 et deuxième tiges transversales à l'intérieur des premier et deuxième logements transversaux, respectivement. Le moyen de réglage 62 est qualifié ici, de moyen de réglage direct. De manière alternative à un bouton rotatif, le moyen de réglage transversal 62 peut être de toute forme permettant de faire coulisser les première 54 et deuxième tiges transversales à l'intérieur des premier et deuxième logements transversaux. De plus, de manière alternative à un coulissement combiné des première 54 et deuxième tiges, le moyen de réglage transversal 62 peut être configuré pour faire coulisser une seule des première 54 et deuxième tiges.

Selon un mode de réalisation alternatif, le réglage permettant de faire coulisser les première 54 et deuxième tiges transversales à l'intérieur des premier et deuxième logements transversaux peut se faire manuellement par exemple, par l'exercice d'une simple pression de l'utilisateur 12. Ainsi, les première 54 et deuxième tiges transversales pénètrent alors à l'intérieur des premier et deuxième logements transversaux s'étendant à la fois au niveau de chacune des portions d'appui 46 et de chacun des côtés de la portion postérieure transversale 24. Dans un second temps, le moyen de réglage transversal 62, peut servir à poursuivre le mouvement initié préalablement et manuellement. De façon alternative, et toujours dans un second temps, le moyen de réglage transversal 62 peut éventuellement ne servir qu'à maintenir dans une position prédéterminée, les première et deuxième tiges transversales à l'intérieur des premier et deuxième logements transversaux. Dans ce dernier cas, le moyen de réglage 62 est qualifié de moyen de réglage indirect.

Pour maintenir les première 54 et deuxième tiges transversales dans une position prédéterminée par rapport aux premier et deuxième logements transversaux, le troisième moyen d'ajustement 40 peut comprendre au moins une saillie formée sur les première 54 et deuxième tiges transversales et au moins un creux formé sur le moyen de réglage transversal 62. La saillie et le creux sont aptes à coopérer ensemble pour maintenir les première 54 et deuxième tiges transversales dans une position prédéterminée par rapport aux premier et deuxième logements transversaux. De manière alternative, la saillie peut être formée sur le moyen de réglage transversal 62 et le creux formé sur la tige sagittale 52. Pour faciliter une utilisation répétée du dispositif d'acquisition 10 sur un même utilisateur 12, les premier 36, deuxième 38 et troisième 40 moyens d'ajustement peuvent comporter une graduation ou tout indicateur permettant de déterminer les positions d'ajustement des premier 36, deuxième 38 et troisième 40 moyens d'ajustement. Les positions d'ajustement sont par exemple une position angulaire d'un bouton, une position linéaire d'un curseur ou encore une position relative d'une tige par rapport à un logement dans lequel la tige coulisse.

A ce titre, la facilité de répétabilité du positionnement des première 32, deuxième 34 et troisième 33 électrodes d'acquisition peut également être autorisée par le fait que, selon un mode de réalisation particulier de l'invention, le support supérieur 30 sur lequel se trouve disposé, directement ou indirectement, les première 32, deuxième 34 et troisième 33 électrodes d'acquisition est apte à être disposé à équidistance de la portion antérieure transversale 22 et de la portion postérieure transversale 24. Ainsi, le moyen de réglage 53 permet de rapprocher ou d'éloigner la portion antérieure transversale 22 de la portion postérieure transversale 24 de façon équidistante par rapport au support supérieur 30 sur lequel se trouve disposé, directement ou indirectement, les première 32, deuxième 34 et troisième 33 électrodes d'acquisition. Ce rapprochement ou cet éloignement est assuré par l'intermédiaire de l'imbrication de la portion antérieure sagittale 18 du support antérieur 14 dans la portion postérieure sagittale 20 du support postérieur 16. Ce positionnement du support supérieur 30 à équidistance de la portion antérieure transversale 22 et de la portion postérieure transversale 24 présente comme avantage de permettre une disposition des première 32, deuxième 34 et troisième 33 électrodes d'acquisition sensiblement en regard des positions C3, C4 et Cz respectivement.

Pour améliorer le maintien du dispositif d'acquisition 10 sur la tête de l'utilisateur 12, le dispositif d'acquisition 10 comprend en outre une portion d'appui 46 configurée pour prendre appui sur une oreille de l'utilisateur 12 lorsque le dispositif d'acquisition 10 est disposé sur la tête de l'utilisateur 12. La portion d'appui 46 est formée au niveau du support antérieur 14. Pour améliorer davantage ce maintien, le dispositif d'acquisition 10 comprend une autre portion d'appui 46 permettant de faire reposer le dispositif d'acquisition 10 sur les deux oreilles de l'utilisateur 12. De plus, la portion d'appui 46 permet de fournir une position de référence pour le positionnement du support antérieur 14 ce qui permet de faciliter le bon positionnement des première 32, deuxième 34 et troisième 33 électrodes d'acquisition au niveau des positions C3 et C4, respectivement, de l'utilisateur 12. En outre, grâce à sa configuration lui permettant de prendre appui sur une oreille de l'utilisateur 12, la portion d'appui 46 peut permettre également de positionner l'une au moins des électrodes de référence ou passive de façon optimale en regard de l'un au moins des processus mastoïde de l'utilisateur,.

Pour faciliter le positionnement du support postérieur 16, le dispositif d'acquisition 10 comprend en outre un évidement 48 apte à indiquer une position de référence du dispositif d'acquisition 10. L'évidement 48 est formé dans le support postérieur 16 de manière à être disposé en regard de la protubérance occipitale externe, de préférence en regard de l'inion situé au niveau de la protubérance occipitale externe de la tête de l'utilisateur 12, ce qui permet à la portion en arceau 11 de s'étendre suivant la plan sagittal Ps. De manière alternative, l'évidement 48 peut prendre tout autre forme permettant au dispositif d'acquisition 10 d'avoir une position de référence pour positionner les première 32, deuxième 34 et troisième 33 électrodes d'acquisition au niveau des positions C3, C4 et Cz, respectivement, de l'utilisateur 12.

La portion d'appui 46 et de l'évidement 48 permettent un positionnement des supports postérieur 16 et antérieur 14 adapté à la morphologie de l'utilisateur 12 garantissant un bon positionnement des première 32, deuxième 34 et troisième 33 électrodes d'acquisition selon les plans sagittal Ps et transversal Pt. De manière alternative, la portion d'appui 46 peut être formée au niveau du support postérieur 16 et l'évidement 48, ou plus généralement un indicateur d'une position de référence, peut être formé au niveau du support antérieur 14 pour garantir également une mise en position des supports antérieur 14 et postérieur 16 adaptée à la morphologie de l'utilisateur 12.

De plus, le dispositif d'acquisition 10 comprend une unité de traitement (non représentée) connectée aux première 32, deuxième 34 et troisième 33 électrodes d'acquisition et, le cas échéant, aux électrodes passive et de référence pour traiter des informations transmises par ces électrodes. L'unité de traitement est logée à l'intérieur du support postérieur 16 et connectée aux première 32, deuxième 34 et troisième 33 électrodes d'acquisition et, le cas échéant, aux électrodes passive et de référence.

Pour permettre une transmission hors du dispositif d'acquisition 10, le dispositif d'acquisition 10 comprend également une unité de transmission des informations traitées par l'unité de traitement. L'unité de transmission comporte un port 64 monté sur le support postérieur 16 pour permettre une transmission filaire. De manière alternative, l'unité de transmission peut être configurée pour permettre une transmission des informations par tout moyen sans fil, par exemple par Wifi, par Bluetooth, par infrarouge, ou encore, par ondes radio.

Pour permettre l'alimentation électrique de l'unité de traitement, de l'unité de transmission et des différentes électrodes, le dispositif d'acquisition 10 comprend également une batterie, par exemple une batterie rechargeable. De manière alternative ou complémentaire à la batterie, le dispositif d'acquisition 10 peut être configuré pour être branché sur secteur. Le dispositif d'acquisition comprend également un interrupteur de mise en tension 66 du dispositif d'acquisition 10.

Selon un mode de réalisation préféré de l'invention, le dispositif d'acquisition 10 comporte des indicateurs visuels de charge de la batterie 68 et de présence d'une connexion 70 entre l'unité de transmission et un dispositif de réception d'information externe au dispositif d'acquisition 10. Ce dispositif externe peut être un téléphone portable connecté avec l'unité de transmission. Dans ce cas, l'unité de traitement est configurée pour transmettre au téléphone portable l'information relative à la qualité du contact physique entre chacune des première 32, deuxième 34 et troisième 33 électrodes d'acquisition et le cuir chevelu de l'utilisateur mais aussi pour permettre ultérieurement au téléphone portable d'émettre un signal d'avertissement rendant compte de ladite qualité du contact physique établi.

Selon un mode de réalisation alternatif de l'invention, pour avertir l'utilisateur 12 du bon positionnement des première 32, deuxième 34 et troisième 33 électrodes d'acquisition, i.e. au niveau des positions C3, C4, et Cz respectivement, l'unité de traitement peut être configurée pour émettre un signal d'avertissement. Ce signal d'avertissement peut être sonore et/ou visuel. De façon complémentaire ou alternative, l'unité de traitement peut être adaptée pour émettre tout autre type de signal d'avertissement et notamment, tactile, olfactif ou encore gustatif. L'émission d'un signal d'avertissement par une unité de traitement intégré au dispositif d'acquisition 10 permet audit dispositif d'acquisition 10 d'être autonome quant à la mise en position des électrodes car aucun appareil externe ne peut être nécessaire pour disposer les électrodes au niveau de leur position souhaitée.

Le dispositif d'acquisition 10 peut être mis en position sur la tête de l'utilisateur 12 au moyen du procédé décrit ci-après. Le support antérieur 14 est tout d'abord déplacé par rapport au support postérieur 16 de manière à ce que la portion annulaire 13 ait une circonférence égale ou supérieure à la circonférence maximale de la tête de l'utilisateur 12 prise dans le plan transversal Pt. Autrement dit, le troisième moyen d'ajustement 40 est actionné de sorte que le dispositif d'acquisition 10 peut être enfilé sur la tête de l'utilisateur 12. Ensuite, le dispositif d'acquisition 10 est positionné sur la tête de l'utilisateur 12 de manière à ce que la portion d'appui 46 prenne appui sur une oreille de l'utilisateur 12 et que l'évidement 48 soit en regard de la protubérance occipitale externe de l'utilisateur 12. Le troisième moyen d'ajustement 40 est actionné à nouveau de manière à ce que la portion annulaire 13 enserre la tête de l'utilisateur 12 suivant le plan transversal Pt. Une fois ce réglage réalisé, le moyen de réglage 62 peut être actionné de sorte à se bloquer, en une position définie et considérée comme optimale, la portion annulaire 13 enserrant la tête de l'utilisateur 12 suivant le plan transversal Pt. Le deuxième moyen d'ajustement 38 est actionné de manière à ce que la portion en arceau 11 enserre la tête de l'utilisateur 12 suivant le plan sagittal Ps. Le premier moyen d'ajustement 36 est ensuite actionné pour positionner les première 32, deuxième 34 et troisième 33 électrodes d'acquisition suivant le plan frontal Pf. De manière alternative, le premier moyen d'ajustement 36 peut être actionné de manière antérieure ou en parallèle de toute étape du procédé décrit ci-dessus.

Pour faciliter la mise en position des première 32, deuxième 34 et troisième 33 électrodes d'acquisition, le procédé de mise en position peut également comprendre la détermination d'une première et d'une deuxième positions d'ajustement des premier 36 et deuxième 38 moyens d'ajustement, respectivement. Les première et deuxième positions d'ajustement sont ensuite mises en mémoire pour pouvoir être réutilisées ultérieurement.

Le dispositif d'acquisition 10 est enlevé de la tête de l'utilisateur et désajusté, par exemple à cause d'une utilisation du dispositif d'acquisition 10 sur un autre utilisateur 12. On entend par « désajusté » le fait que les première et deuxième positions d'ajustement sont différentes des première et deuxième positions d'ajustement mises en mémoire.

Le dispositif d'acquisition 10 est ensuite disposé à nouveau sur la tête de l'utilisateur 12 mais en actionnant les premier 36 et deuxième 38 moyens d'ajustement de manière à les disposer selon les première et deuxième positions d'ajustement mises en mémoire. La mise en position des première 32, deuxième 34 et troisième 33 électrodes d'acquisition au niveau des positions C3, C4 et Cz est ainsi facilitée et accélérée. La détermination ou la quantification des positions d'ajustement est rendue possible car l'ajustement de la position des première 32, deuxième 34 et troisième électrodes d'acquisition est décomposé pour chacun des plans sagittal Ps, frontal Pf et transversal Pt.

Par ailleurs, le dispositif d'acquisition 10 peut être utilisé entre autres dans un procédé de caractérisation du sommeil de l'utilisateur 12 présenté ci-dessous.

A l'état endormi, la différence de potentiel électrique entre les première 32, deuxième 34 et troisième 33 électrodes d'acquisition et, le cas échéant, les électrodes de référence et passive pour obtenir le signal d'activité cérébrale représentatif des ondes cérébrales de l'utilisateur 12. Ce signal d'activité cérébrale est amplifié et numérisé par l'unité de traitement.

Dans la plage de fréquences de 9 à 16 Hz du signal d'activité cérébrale en phase de sommeil léger (phase appelée stade N2 du sommeil NREM, de l'anglais « *non rapid eye movement* ») (De Gennaro & Ferrara, 2003; De Gennaro, Ferrara, Curcio, & Cristiani, 2001), l'unité de traitement identifie la plage réduite de fuseaux de sommeil de l'utilisateur 12. Cette plage réduite de fuseaux de sommeil comprend les fréquences d'ondes cérébrales de l'utilisateur endormi présentant une amplitude supérieure à 15 µV et une durée comprise entre 0,5 seconde et 2 secondes.

A l'état éveillé, la différence de potentiel électrique entre les première 32, deuxième 34 et troisième 33 électrodes d'acquisition et, le cas échéant, les électrodes de référence et passive est mesurée par le dispositif d'acquisition 10 pour obtenir le signal d'activité cérébrale représentatif des ondes cérébrales de l'utilisateur 12. Ce signal d'activité cérébrale est amplifié et numérisé par l'unité de traitement.

Dans la plage de fréquences correspondant à la plage réduite de fuseaux de sommeil, l'unité de traitement compare à un seuil au moins l'un des deux paramètres suivants que sont l'amplitude ou la densité.

Lorsque le paramètre du signal d'activité cérébrale dans la plage de fréquences correspondant à la plage réduite de fuseaux de sommeil atteint ou dépasse le seuil, un signal d'avertissement est émis. Ce signal d'avertissement peut être sonore et/ou visuel. De façon complémentaire ou alternative, l'unité de traitement peut être adaptée pour émettre, directement ou par l'intermédiaire d'un dispositif externe, tout autre type de signal d'avertissement et notamment, tactile, olfactif ou encore gustatif.

L'utilisateur 12 est ainsi averti de l'émission d'ondes cérébrales favorisant la qualité du sommeil.

A partir de cette caractérisation du sommeil, un traitement de Neurofeedback peut être mis en place pour améliorer la qualité du sommeil de l'utilisateur 12. On appelle Neurofeedback une technologie permettant d'amener un individu à agir sur une activité biologique, ici nerveuse ou neurologique, par un traitement de rétrocontrôle. Ce traitement de Neurofeedback consiste par exemple en un entraînement de l'individu pour l'aider à se relaxer et l'inciter à modifier son activité cérébrale. Le traitement de Neurofeedback comprend alors un entrainement visant à inciter l'utilisateur 12 à produire les ondes cérébrales souhaitées. L'entrainement comprend une ou plusieurs séquences de relaxation et d'exercices récompensant l'émission des ondes cérébrales souhaitées par des signaux d'avertissement particuliers.

Au cours de l'entrainement, le seuil peut être augmenté pour faire progresser l'utilisateur 12 dans la production d'ondes cérébrales appropriées. Au cours de l'entrainement, le seuil peut aussi être abaissé pour faciliter la tâche d'un utilisateur 12 qui serait peu performant.

Bien entendu, la présente invention n'est pas limitée aux exemples et au mode de réalisation décrits et représentés, mais elle est susceptible de nombreuses variantes accessibles à l'homme de l'art.

Par exemple, le dispositif d'acquisition 10 peut s'affranchir du support supérieur 30. Dans ce cas, la portion en arceau 11 s'étend suivant le plan frontal Pf et intègre les première 32, deuxième 34 et troisième 33 électrodes d'acquisition et le premier moyen d'ajustement 36.

De manière alternative, le support supérieur 30 peut être monté sur la portion antérieure sagittale 18 du support antérieur 14 au lieu d'être monté sur le support postérieur 16, au niveau de la portion postérieure sagittale 20. Dans ce cas, la portion d'appui 46 est formée au niveau de la portion postérieure transversale 24 et configurée pour que la portion antérieur transversale 22 coulisse par rapport à la portion d'appui 46. Ainsi, le déplacement de la portion antérieur transversale 22 par rapport à la portion postérieure transversale 24 permet l'ajustement de la position des première 32, deuxième 34 et troisième 33 électrodes d'acquisition suivant le plan transversal Pt.

De plus, de manière alternative à une configuration où les supports antérieur 14 et postérieur 16 forment à la fois la portion annulaire 13 et la portion en arceau 11, le dispositif d'acquisition 10 peut être configuré de sorte que le support antérieur 14 est apte à coopérer avec le support postérieur 16 pour former la portion annulaire 13 destinée à enserrer la tête de l'utilisateur 12 suivant le plan transversal Pt ou apte à coopérer avec le support postérieur 16 pour enserrer la tête de l'utilisateur 12 suivant le plan sagittal Ps. Dans ces deux dernières configurations, le dispositif d'acquisition 10 peut comprendre des supports supplémentaires coopérant avec les supports antérieur 14 et postérieur 16 pour réaliser le maintien du dispositif d'acquisition 10 sur la tête de l'utilisateur 12.

Par ailleurs, de manière alternative ou complémentaire au mode de réalisation présenté sur les figures 2 à 4, le dispositif d'acquisition 10 peut prévoir d'autres moyens d'ajustement de la position des première 32, deuxième 34 et troisième 33 électrodes d'acquisition. Par exemple, le support supérieur 30 peut être déplaçable par rapport à la portion postérieure sagittale 20 selon une direction comprise dans le plan sagittal Ps ou déplaçable en rotation autour d'un axe s'étendant transversalement au plan transversal Pt de l'utilisateur 12. De plus, les premier 42 et deuxième 44 curseurs peuvent être déplaçables par rapport au support supérieur 30 selon une direction comprise dans le plan sagittal Ps de manière à pouvoir ajuster la position des première 32, deuxième 34 et troisième 33 électrodes d'acquisition selon le plan sagittal Ps et le plan frontal Pf.

De plus, de manière alternative à un actionnement indépendant des premier 36, deuxième 38 et troisième 40 moyens d'ajustement, seuls les premier 36 et troisième 40 moyens d'ajustement ou seuls les deuxième 38 et troisième 40 moyens d'ajustement peuvent être configurés pour être actionnés indépendamment.

La présente description est donnée à titre d'exemple, et n'est pas limitative de l'invention. En particulier, l'invention concerne l'ajustement de la position d'au moins une électrode au niveau d'au moins une quelconque des positions du système international 10/20, et ne se limite donc pas au contexte de l'étude des troubles du sommeil ou des troubles de déficit de l'attention avec ou sans hyperactivité (TDAH) avec comme positions d'intérêt C3, C4 et/ou Cz.

Elle s'étend par exemple aux contextes de l'étude des troubles de la mémoire avec d'autres positions d'intérêt telles que les positions CPz, FT7, et/ou T7, ou T8. Des supports complémentaires peuvent être requis pour supporter la ou les électrodes en ses positions, reliés directement ou indirectement à l'un et/ou l'autre des supports postérieurs 14, antérieur 16 et supérieur 30.

## Revendications

1. Dispositif d'acquisition (10) destiné à être positionné sur la tête d'un utilisateur (12) pour réaliser un encéphalogramme, la tête de l'utilisateur (12) étant définie selon un plan transversal (Pt), un plan sagittal (Ps) et un plan frontal (Pf), le dispositif comprenant :
- au moins une électrode d'acquisition (32, 34, 33) configurée pour être positionnée au contact de la tête de l'utilisateur (12) au niveau d'au moins une quelconque des positions du système international 10/20 de manière à acquérir au moins un signal d'activité cérébrale représentatif des ondes cérébrales de l'utilisateur (12);
- au moins un premier (36, 38, 40) et un deuxième (36, 38, 40) moyens d'ajustement de la position de l'électrode d'acquisition (32, 34, 33) suivant un premier et un deuxième plans, respectivement ;
dans lequel les premier et deuxième plans sont différents et choisis parmi au moins les plans transversal (Pt) et sagittal (Ps) de la tête de l'utilisateur (12),
dans lequel lesdits premier (36, 38, 40) et deuxième (36, 38, 40) moyens d'ajustement de la position de l'électrode d'acquisition (32, 34, 33) sont actionnables indépendamment l'un de l'autre
dans lequel le dispositif d'acquisition (10) est configuré sous la forme d'un casque, le casque comprenant une portion annulaire (13) pour enserrer la tête de l'utilisateur (12) dans le plan transversal (Pt) et une portion en arceau (11) reliée au niveau d'extrémités à la portion annulaire (13) pour pouvoir faire reposer le dispositif d'acquisition sur la tête de l'utilisateur (12), l'électrode d'acquisition étant montée sur l'arceau,
dans lequel les premier et deuxième moyens d'ajustement de la position de l'électrode d'acquisition (32, 34, 33) permettent également d'adapter le dispositif d'acquisition (10) à la tête de l'utilisateur (12) suivant les plans sagittal (Ps) et transversal (Pt),
dans lequel le casque est rigide tel que la position de l'électrode d'acquisition ne peut être modifiée que par actionnement de l'un ou plusieurs des premier et deuxième moyens d'ajustement.

2. Dispositif d'acquisition (10) selon la revendication 1, lequel comprenant en outre :
- un support postérieur (16) ;
- un support antérieur (14) apte à coopérer avec le support postérieur (16) pour former la portion annulaire (13) et apte à coopérer avec le support postérieur (16) pour former la portion en arceau (11) destinée à enserrer la tête de l'utilisateur (12) suivant le plan sagittal (Ps),
- un support supérieur (30) sur lequel est montée l'électrode d'acquisition (32, 34, 33), le support supérieur (30) étant monté sur le support antérieur (14) ou postérieur (16).

3. Dispositif d'acquisition (10) selon la revendication 2, comprenant en outre un troisième moyen d'ajustement (36, 38, 40) de la position de l'électrode d'acquisition (32, 34, 33) suivant un troisième plan choisi parmi les plans transversal (Pt), sagittal (Ps) et frontal (Pf) de la tête de l'utilisateur (12), les premier, deuxième et troisième plans étant différents, les premier (36, 38, 40), deuxième (36, 38, 40) et troisième (36, 38, 40) moyens d'ajustement de la position de l'électrode d'acquisition (32, 34,33) étant de préférence actionnables indépendamment les uns des autres.

4. Dispositif d'acquisition (10) selon la revendication 3, dans lequel le support supérieur (30) est associé au support postérieur (16), dans lequel :
- le premier moyen d'ajustement (36) est apte à déplacer l'électrode d'acquisition (32, 34, 33) par rapport au support supérieur (30) selon une première direction comprise dans le plan frontal (Pf) ;
- le deuxième moyen d'ajustement (38) est apte à déplacer le support antérieur (14) par rapport au support postérieur (16) selon une deuxième direction comprise dans le plan sagittal (Ps); et
- le troisième moyen d'ajustement (40) est apte à déplacer le support antérieur (14) par rapport au support postérieur (16) selon une troisième direction comprise dans le plan transversal (Pt)

5. Dispositif d'acquisition (10) selon la revendication 4, dans lequel le premier moyen d'ajustement (36) comprend un curseur (42, 44) apte à coulisser selon la première direction comprise dans le plan frontal (Pf) dans un logement frontal formé dans le support supérieur (30), l'au moins une électrode d'acquisition (32, 34) étant montée sur le curseur (42, 44).

6. Dispositif d'acquisition (10) selon la revendication 4 ou 5, dans lequel les deuxième (38) et troisième (40) moyens d'ajustement comprennent :
- une tige (50, 54) formée sur le support antérieur (14) ;
- un logement (52) formé dans le support postérieur (16) et dans lequel la tige est apte à coulisser ; et
- un moyen de réglage direct ou indirect (53, 62) de la position de la tige (50, 54) montée sur le support postérieur (16) et apte à coopérer avec la tige (50, 54) pour faire coulisser la tige (50, 54) à l'intérieur du logement (52) de manière à déplacer le support antérieur (14) par rapport au support postérieur (16).

7. Dispositif d'acquisition (10) selon l'une des revendications 4 à 6, dans lequel les deuxième (38) et troisième (40) moyens d'ajustement comportent au moins une saillie et au moins un creux formés sur la tige (50, 54) ou le moyen de réglage direct ou indirect (53, 62), respectivement, la saillie et le creux étant aptes à coopérer ensemble pour maintenir la tige (50, 54) dans une position prédéterminée par rapport au logement (52).

8. Dispositif d'acquisition (10) selon l'une quelconque des revendications 2 à 7, comprenant une première (32), et/ou une deuxième (34) et/ou une troisième (33) électrodes d'acquisition configurées pour être positionnées au contact de la tête de l'utilisateur (12) au niveau de l'une des positions C3, C4 et/ou Cz, respectivement, du système international 10/20, et dans lequel les première (32), et/ou deuxième (34) et/ou troisième (33) électrodes d'acquisition sont montées sur le support supérieur (30).

9. Dispositif d'acquisition (10) selon la revendication 8, comprenant en outre une électrode d'acquisition configurée pour être positionnée au contact de la tête de l'utilisateur (12) au niveau de la position CPz du système international 10/20, et/ou une électrode d'acquisition configurée pour être positionnées au contact de la tête de l'utilisateur (12) au niveau de la position FT7 du système international 10/20.

10. Dispositif d'acquisition (10) selon la revendication 8, comprenant en outre une électrode d'acquisition configurée pour être positionnée au contact de la tête de l'utilisateur (12) au niveau de la position T3 du système international 10/20, et/ou une électrode d'acquisition configurée pour être positionnées au contact de la tête de l'utilisateur (12) au niveau de la position T4 du système international 10/20.

11. Dispositif d'acquisition (10) selon l'une quelconque des revendications 2 à 10, dans lequel le support antérieur (14) comprend une portion d'appui (46) configurée pour prendre appui sur une oreille de l'utilisateur (12) lorsque le dispositif d'acquisition (10) est disposé sur la tête de l'utilisateur (12).

12. Dispositif d'acquisition (10) selon la revendication 11, dans lequel la portion d'appui (46) comprend au moins une électrode de référence configurée pour être positionnée au contact de la tête de l'utilisateur (12), de préférence en regard de l'un des processus mastoïde de l'utilisateur (12).

13. Dispositif d'acquisition (10) selon l'une quelconque des revendications 2 à 12, dans lequel le support supérieur (30) est monté sur le support postérieur (16), le support postérieur (16) comprenant un évidement (48) apte à indiquer une position de référence du support postérieur (16), l'évidement (48) étant de préférence configuré pour être disposé en regard de la protubérance occipitale externe, de préférence au niveau de l'inion, de la tête de l'utilisateur (12).

14. Dispositif d'acquisition (10) selon l'une quelconque des revendications 2 à 13, comprenant en outre :
- une unité de traitement connectée à l'au moins une électrode d'acquisition (32, 34, 33) et, le cas échéant, à l'au moins une électrode de référence pour traiter des informations transmises par l'au moins une électrode d'acquisition (32, 34, 33) et l'au moins une électrode de référence ;
- une unité de transmission des informations traitées par l'unité de traitement.

15. Procédé de mise en position d'un dispositif d'acquisition (10) sur la tête d'un utilisateur (12) pour réaliser un encéphalogramme, la tête de l'utilisateur (12) étant définie selon un plan transversal (Pt), un plan sagittal (Ps) et un plan frontal (Pf), le procédé comprenant les étapes consistant à :
- fournir un dispositif d'acquisition (10) selon l'une quelconque des revendications 3 à 14 ;
- disposer le dispositif d'acquisition (10) sur la tête de l'utilisateur (12) ;
- actionner le troisième moyen d'ajustement (40) pour enserrer la tête de l'utilisateur (12),
- actionner le deuxième moyen d'ajustement (38) de manière à placer le support supérieur (30) équidistant d'une extrémité distale de la portion antérieure transversale (22) et d'une d'extrémité distale de la portion postérieure transversale (24),
- actionner le premier moyen d'ajustement (36) pour ajuster la position de l'au moins une électrode d'acquisition (32, 34, 33) suivant le plan frontal (Pf) ;
dans lequel les étapes d'actionnement des premier (36, 38, 40) et deuxième (36, 38, 40) moyens d'ajustement sont réalisées de manière indépendante.

16. Procédé de mise en position selon la revendication 15, comprenant en outre les étapes consistant à :
- déterminer une première et une deuxième positions d'ajustement des premier (36, 38, 40) et deuxième (36, 38, 40) moyens d'ajustement, respectivement ;
- mettre en mémoire les première et deuxième positions d'ajustement ;
- actionner les premier (36, 38, 40) et deuxième (36, 38, 40) moyens d'ajustement de manière à les positionner dans des positions d'ajustement différentes des première et deuxième positions d'ajustement ;
- enlever le dispositif d'acquisition (10) de la tête de l'utilisateur (12) ;
- disposer à nouveau le dispositif d'acquisition (10) sur la tête de l'utilisateur (12) ;
- actionner les premier (36, 38, 40) et deuxième (36, 38, 40) moyens d'ajustement de manière à les disposer selon les première et deuxième positions d'ajustement.

17. Procédé de mise en position selon la revendication 15 ou 16, dans lequel le dispositif d'acquisition (10) est un dispositif d'acquisition (10) selon l'une quelconque des revendications 2 à 14 prise en combinaison avec les revendications 4, 11 et 13, dans lequel l'étape consistant à disposer le dispositif d'acquisition (10) sur la tête de l'utilisateur (12) comprend les étapes consistant à :
- déplacer le support antérieur (14) par rapport au support postérieur (16) de manière à ce que la portion en arceau (11) ait une circonférence égale ou supérieure à la circonférence maximale de la tête de l'utilisateur (12) prise dans le plan transversal (Pt) ;
- positionner le dispositif d'acquisition (10) sur la tête de l'utilisateur (12) de manière à ce que la portion d'appui (46) prenne appui sur une oreille de l'utilisateur (12) ;
- positionner le dispositif d'acquisition (10) sur la tête de l'utilisateur (12) de manière à ce que l'évidement (48) du support postérieur (16) soit en regard de la protubérance occipitale externe de la tête de l'utilisateur (12).

## Patentansprüche

1. Erfassungsvorrichtung (10), die dazu bestimmt ist, auf dem Kopf eines Benutzers (12) positioniert zu werden, um ein Enzephalogramm durchzuführen, wobei der Kopf des Benutzers (12) gemäß einer Querebene (Pt), einer Sagittalebene (Ps) und einer Frontalebene (Pf) definiert ist, wobei die Vorrichtung umfasst:
- mindestens eine Erfassungselektrode (32, 34, 33), die konfiguriert ist, um in Kontakt mit dem Kopf des Benutzers (12) an mindestens einer der Positionen des internationalen 10/20-Systems positioniert zu werden, um mindestens ein Gehirnaktivitätssignal zu erfassen, das repräsentativ für die Gehirnwellen des Benutzers (12) ist;
- mindestens ein erstes (36, 38, 40) und ein zweites (36, 38, 40) Mittel zum Einstellen der Position der Erfassungselektrode (32, 34, 33) in einer ersten bzw. einer zweiten Ebene;
wobei die erste und die zweite Ebene unterschiedlich sind und mindestens aus der Transversalebene (Pt) und der Sagittalebene (Ps) des Kopfes (12) des Benutzers ausgewählt sind,
wobei das erste (36, 38, 40) und das zweite (36, 38, 40) Mittel zum Einstellen der Position der Erfassungselektrode (32, 34, 33) unabhängig voneinander betreibbar sind
wobei die Erfassungsvorrichtung (10) in Form eines Helms konfiguriert ist, wobei der Helm einen ringförmigen Abschnitt (13) zum Umschließen des Kopfes (12) des Benutzers in der Querebene (Pt) und einen Bügelabschnitt (11) aufweist, der an den Enden mit dem ringförmigen Abschnitt (13) verbunden ist, um die Erfassungsvorrichtung auf dem Kopf (12) des Benutzers aufsetzen zu können, wobei die Erfassungselektrode auf dem Bügel montiert ist,
bei der die ersten und zweiten Mittel zur Einstellung der Position der Erfassungselektrode (32, 34, 33) es auch ermöglichen, die Erfassungsvorrichtung (10) an den Kopf des Benutzers (12) entlang der Sagittalebene (Ps) und der Transversalebene (Pt) anzupassen,
wobei der Helm starr und so konfiguriert ist, dass die Position der Erfassungselektrode nur durch Betätigung eines oder mehrerer der ersten und zweiten Einstellmittel verändert werden kann.

2. Eine Erfassungsvorrichtung (10) nach Anspruch 1, die ferner umfasst:
- a hintere Stütze (16);
- eine vordere Stütze (14), die dazu geeignet ist, mit der hinteren Stütze (16) zusammenzuwirken, um den ringförmigen Abschnitt (13) zu bilden, und die dazu geeignet ist, mit der hinteren Stütze (16) zusammenzuwirken, um den Bogenabschnitt (11) zu bilden, der dazu bestimmt ist, den Kopf (12) des Benutzers in der Sagittalebene (Ps) zu umschließen,
- einen oberen Träger (30), auf dem die Erfassungselektrode (32, 34, 33) montiert ist, wobei der obere Träger (30) auf dem vorderen (14) oder hinteren (16) Träger montiert ist.

3. Erfassungsvorrichtung (10) nach Anspruch 2, die ferner ein drittes Mittel (36, 38, 40) zum Einstellen der Position der Erfassungselektrode (32, 34, 33) entlang einer dritten Ebene umfasst, die aus der Querebene (Pt), der Sagittalebene (Ps) und der Frontalebene (Pf) des Kopfes (12) des Benutzers ausgewählt wird, wobei die erste, zweite und dritte Ebene unterschiedlich sind, wobei die ersten (36, 38, 40), zweiten (36, 38, 40) und dritten (36, 38, 40) Mittel zur Einstellung der Position der Erfassungselektrode (32, 34, 33) vorzugsweise unabhängig voneinander betrieben werden können.

4. Erfassungsvorrichtung (10) nach Anspruch 3, wobei die obere Halterung (30) mit der hinteren Halterung (16) verbunden ist, wobei:
- die erste Einstelleinrichtung (36) in der Lage ist, die Erfassungselektrode (32, 34, 33) relativ zu der oberen Halterung (30) in einer ersten Richtung in der Frontalebene (Pf) zu verschieben;
- das zweite Einstellmittel (38) angepasst ist, um die vordere Stütze (14) relativ zur hinteren Stütze (16) in eine zweite Richtung in der Sagittalebene (Ps) zu bewegen; und
- das dritte Einstellmittel (40) in der Lage ist, die vordere Stütze (14) in Bezug auf die hintere Stütze (16) in einer dritten Richtung in der Querebene (Pt) zu verschieben.

5. Erfassungsvorrichtung (10) nach Anspruch 4, bei der das erste Einstellmittel (36) einen Läufer (42, 44) umfasst, der in der ersten Richtung gleiten kann, die in der Frontalebene (Pf) in einer in der oberen Halterung (30) ausgebildeten Frontaufnahme enthalten ist, wobei die mindestens eine Erfassungselektrode (32, 34) auf dem Läufer (42, 44) montiert ist.

6. Erfassungsvorrichtung (10) nach Anspruch 4 oder 5, wobei die zweiten (38) und dritten (40) Einstellmittel umfassen:
- eine Stange (50, 54), die an der vorderen Stütze (14) ausgebildet ist;
- ein Gehäuse (52), das in der hinteren Halterung (16) ausgebildet ist und in dem die Stange verschiebbar ist; und
- Mittel (53, 62) zur direkten oder indirekten Einstellung der Position der Stange (50, 54), die an der hinteren Halterung (16) angebracht sind und mit der Stange (50, 54) zusammenwirken können, um die Stange (50, 54) im Inneren des Gehäuses (52) so zu verschieben, dass die vordere Halterung (14) relativ zur hinteren Halterung (16) bewegt wird.

7. Erfassungsvorrichtung (10) nach einem der Ansprüche 4 bis 6, wobei das zweite (38) und das dritte (40) Einstellmittel mindestens einen Vorsprung und mindestens eine Aussparung aufweisen, die an der Stange (50, 54) bzw. dem direkten oder indirekten Einstellmittel (53, 62) ausgebildet sind, wobei der Vorsprung und die Aussparung zusammenwirken können, um die Stange (50, 54) in einer vorbestimmten Position relativ zum Gehäuse (52) zu halten.

8. Erfassungsvorrichtung (10) nach einem der Ansprüche 2 bis 7, umfassend eine erste (32) und/oder eine zweite (34) und/oder eine dritte (33) Erfassungselektrode, die so konfiguriert ist, dass sie in Kontakt mit dem Kopf (12) des Benutzers an einer der Positionen C3, C4 und/oder Cz des internationalen 10/20-Systems positioniert werden kann, und wobei die erste (32) und/oder die zweite (34) und/oder die dritte (33) Erfassungselektrode auf dem oberen Träger (30) angebracht sind.

9. Erfassungsvorrichtung (10) nach Anspruch 8, ferner umfassend eine Erfassungselektrode, die konfiguriert ist, um in Kontakt mit dem Kopf (12) des Benutzers an der CPz-Position des internationalen 10/20-Systems positioniert zu werden, und/oder eine Erfassungselektrode, die konfiguriert ist, um in Kontakt mit dem Kopf (12) des Benutzers an der FT7-Position des internationalen 10/20-Systems positioniert zu werden.

10. Erfassungsvorrichtung (10) nach Anspruch 8, weiterhin umfassend eine Erfassungselektrode, die konfiguriert ist, um in Kontakt mit dem Kopf (12) des Benutzers an der Position T3 des internationalen 10/20-Systems positioniert zu werden, und/oder eine Erfassungselektrode, die konfiguriert ist, um in Kontakt mit dem Kopf (12) des Benutzers an der Position T4 des internationalen 10/20-Systems positioniert zu werden.

11. Erfassungsvorrichtung (10) nach einem der Ansprüche 2 bis 10, wobei die vordere Stütze (14) einen Stützabschnitt (46) umfasst, der so konfiguriert ist, dass er auf dem Ohr (12) eines Benutzers ruht, wenn die Erfassungsvorrichtung (10) auf dem Kopf (12) des Benutzers angeordnet ist.

12. Erfassungsvorrichtung (10) nach Anspruch 11, wobei der Stützabschnitt (46) mindestens eine Referenzelektrode umfasst, die so konfiguriert ist, dass sie in Kontakt mit dem Kopf (12) des Benutzers positioniert werden kann, vorzugsweise in Bezug auf einem der knöchernen Vorsprünge des Schläfenbeins (Processus mastoideus) (12) des Benutzers.

13. Erfassungsvorrichtung (10) nach einem der Ansprüche 2 bis 12, bei der die obere Halterung (30) auf der hinteren Halterung (16) montiert ist, wobei die hintere Halterung (16) eine Aussparung (48) aufweist, die geeignet ist, eine Referenzposition der hinteren Halterung (16) anzuzeigen, wobei die Aussparung (48) vorzugsweise so konfiguriert ist, dass sie der äußeren okzipitalen Vorwölbung des Kopfes (12) des Benutzers, vorzugsweise am Hinterhaupthöcker (Inion), zugewandt ist.

14. Eine Erfassungsvorrichtung (10) gemäß einem der Ansprüche 2 bis 13, ferner umfassend:
- eine Verarbeitungseinheit, die mit der mindestens einen Erfassungselektrode (32, 34, 33) und gegebenenfalls mit der mindestens einen Referenzelektrode verbunden ist, zur Verarbeitung von Informationen, die von der mindestens einen Erfassungselektrode (32, 34, 33) und der mindestens einen Referenzelektrode übertragen werden;
- eine Einheit zur Übertragung der von der Verarbeitungseinheit verarbeiteten Informationen.

15. Verfahren zum Positionieren einer Erfassungsvorrichtung (10) auf dem Kopf eines Benutzers (12), um ein Enzephalogramm durchzuführen, wobei der Kopf des Benutzers (12) gemäß einer Querebene (Pt), einer Sagittalebene (Ps) und einer Frontalebene (Pf) definiert ist, wobei das Verfahren die folgenden Schritte umfasst::
- Bereitstellens eines Erfassungsgeräts (10) gemäß einem der Ansprüche 3 bis 14;
- Setzen des Erfassungsgeräts (10) auf den Kopf des Benutzers (12);
- Betätigen des dritten Einstellmittels (40), um den Kopf des Benutzers zu klemmen (12),
- Betätigen des zweiten Einstellmittels (38), um die obere Halterung (30) in gleicher Entfernung von einem distalen Ende des vorderen Querteils (22) und einem distalen Ende des hinteren Querteils (24) zu positionieren,
- Betätigen des ersten Einstellmittels (36), um die Position der mindestens einen Erfassungselektrode (32, 34, 33) entsprechend der Frontalebene (Pf) einzustellen;
wobei die Schritte des Betätigens der ersten (36, 38, 40) und zweiten (36, 38, 40) Einstellmittel unabhängig voneinander durchgeführt werden.

16. Verfahren zur Positionierung nach Anspruch 15, das ferner die folgenden Schritte umfasst:
- Bestimmung der ersten und zweiten Einstellposition des ersten (36, 38, 40) bzw. zweiten (36, 38, 40) Einstellmittels;
- Speichern der ersten und zweiten Einstellposition;
- Betätigung der ersten (36, 38, 40) und zweiten (36, 38, 40) Einstellmittel, um sie in andere Einstellpositionen als die erste und zweite Einstellposition zu bringen;
- Abnehmen des Erfassungsgeräts (10) vom Kopf (12) des Benutzers;
- Erneutes Setzen des Erfassungsgeräts (10) auf den Kopf (12) des Benutzers;
- Betätigung der ersten (36, 38, 40) und zweiten (36, 38, 40) Einstellmittel, um sie entsprechend der ersten und zweiten Einstellposition anzuordnen.

17. Verfahren zum Positionieren nach Anspruch 15 oder 16, wobei die Erfassungsvorrichtung (10) eine Erfassungsvorrichtung (10) nach einem der Ansprüche 2 bis 14 in Kombination mit den Ansprüchen 4, 11 und 13 ist, wobei der Schritt des Anordnens der Erfassungsvorrichtung (10) auf dem Kopf (12) des Benutzers die folgenden Schritte umfasst::
- Bewegen der vorderen Stütze (14) relativ zur hinteren Stütze (16), so dass der Bogenteil (11) einen Umfang hat, der gleich oder größer ist als der maximale Umfang des Kopfes (12) des Benutzers, gemessen in der Querebene (Pt);
- Positionieren der Erfassungsvorrichtung (10) auf dem Kopf des Benutzers (12), so dass der Stützabschnitt (46) auf einem Ohr des Benutzers (12) ruht;
- Positionieren der Erfassungsvorrichtung (10) auf dem Kopf (12) des Benutzers, so dass die Aussparung (48) der hinteren Stütze (16) der äußeren okzipitalen Ausstülpung des Kopfes (12) des Benutzers zugewandt ist.

## Claims

1. Acquisition device (10) intended to be positioned on the head of a user (12) in order to perform an encephalogram, the head of the user (12) being defined in a transverse plane (Pt), a sagittal plane (Ps) and a frontal plane (Pf), the device comprising:
- at least one acquisition electrode (32, 34, 33) configured in order to be positioned in contact with the head of the user (12) at at least any one of the positions of the international 10-20 system, so as to acquire at least one brain activity signal representative of the brain waves of the user (12);
- at least one first (36, 38, 40) and one second (36, 38, 40) means of adjusting the position of the acquisition electrode (32, 34, 33) in a first and a second plane, respectively;
in which the first and second planes are different and chosen from at least the transverse (Pt) and sagittal (Ps) planes of the head of the user (12),
in which said first (36, 38, 40) and second (36, 38, 40) means of adjusting the position of the acquisition electrode (32, 34, 33) are capable of being actuated independently of one another
in which the acquisition device (10) is in the form of a headset, the headset comprising an annular portion (13) to tightly fit around the head of the user (12) in the transverse plane (Pt) and an arched portion (11) connected at ends to the annular portion (13) in order to be able to rest the acquisition device on the head of the user (12), the acquisition electrode being mounted on the arched portion,
in which the first and second means of adjusting the position of the acquisition electrode (32, 34, 33) also enable to adapt the acquisition device (10) to the head of the user (12) along the sagittal (Ps) and transverse (Pt) planes,
in which the headset is rigid so that the position of the acquisition electrode can only by modified by actuating one or several of the first and second means of adjusting.

2. Acquisition device (10) according to claim 1, said device also comprising:
- a posterior support (16);
- an anterior support (14) capable of engaging with the posterior support (16) in order to form the annular portion (13) and capable of cooperating with the posterior support (16) in order to form the arched portion (11) intended to tightly fit around the head of the user (12) in the sagittal plane (Ps),
- an upper support (30) on which is mounted the acquisition electrode (32, 34, 33), the upper support (30) being mounted on the anterior (14) or posterior (16) support.

3. Acquisition device (10) according to claim 2, also comprising a third means (36, 38, 40) of adjusting the position of the acquisition electrode (32, 34, 33) in a third plane chosen from the transverse (Pt), sagittal (Ps) and frontal (Pf) planes of the head of the user (12), the first, second and third planes being different, the first (36, 38, 40), second (36, 38, 40) and third (36, 38, 40) means of adjusting the position of the acquisition electrode (32, 34, 33) preferably being capable of actuation independently of one another.

4. Acquisition device (10) according to claim 3, in which the upper support (30) is combined with the posterior support (16), in which:
- the first adjustment means (36) is capable of displacing the acquisition electrode (32, 34, 33) with respect to the upper support (30) in a first direction comprised within the frontal plane (Pf);
- the second adjustment means (38) is capable of displacing the anterior support (14) with respect to the posterior support (16) in a second direction comprised within the sagittal plane (Ps); and
- the third adjustment means (40) is capable of displacing the anterior support (14) with respect to the posterior support (16) in a third direction comprised within the transverse plane (Pt).

5. Acquisition device (10) according to claim 4, in which the first adjustment means (36) comprises a slider (42, 44) capable of sliding in the first direction comprised within the frontal plane (Pf) in a frontal housing formed in the upper support (30), the at least one acquisition electrode (32, 34) being mounted on the slider (42, 44).

6. Acquisition device (10) according to claim 4 or 5, in which the second (38) and third (40) adjustment means comprise:
- a rod (50, 54) formed on the anterior support (14);
- a housing (52) formed in the posterior support (16) in which the rod is capable of sliding; and
- an indirect or direct means (53, 62) of adjusting the position of the rod (50, 54) mounted on the posterior support (16) and capable of cooperating with the rod (50, 54) in order to slide the rod (50, 54) inside the housing (52) so as to displace the anterior support (14) with respect to the posterior support (16).

7. Acquisition device (10) according to one of claims 4 to 6, in which the second (38) and third (40) adjustment means comprise at least one projection and at least one recess formed on the rod (50, 54) or the direct or indirect adjustment means (53, 62), respectively, the projection and the recess being capable of cooperating with one another in order to hold the rod (50, 54) in a predetermined position with respect to the housing (52).

8. Acquisition device (10) according to any one of claims 2 to 7, comprising a first (32), and/or a second (34) and/or a third (33) acquisition electrodes configured in order to be positioned in contact with the head of the user (12) at one of the positions C3, C4 and/or Cz, respectively, of the international 10-20 system, and in which the first (32), and/or second (34) and/or third (33) acquisition electrodes are mounted on the upper support (30).

9. Acquisition device (10) according to claim 8, also comprising an acquisition electrode configured in order to be positioned in contact with the head of the user (12) at the position CPz of the international 10-20 system, and/or an acquisition electrode configured in order to be positioned in contact with the head of the user (12) at the position FT7 of the international 10-20 system.

10. Acquisition device (10) according to claim 8, also comprising an acquisition electrode configured in order to be positioned in contact with the head of the user (12) at the position T3 of the international 10-20 system, and/or an acquisition electrode configured in order to be positioned in contact with the head of the user (12) at the position T4 of the international 10-20 system.

11. Acquisition device (10) according to any one of claims 2 to 10, in which the anterior support (14) comprises a bearing portion (46) configured in order to bear on an ear of the user (12) when the acquisition device (10) is arranged on the head of the user (12).

12. Acquisition device (10) according to claim 11, in which the bearing portion (46) comprises at least one reference electrode configured in order to be positioned in contact with the head of the user (12), preferably facing one of the mastoid processes of the user (12).

13. Acquisition device (10) according to any one of claims 2 to 12, in which the upper support (30) is mounted on the posterior support (16), the posterior support (16) comprising a hollow (48) capable of indicating a reference position of the posterior support (16), the hollow (48) preferably being configured in order to be arranged facing the external occipital protuberance, preferably at the inion, of the head of the user (12).

14. Acquisition device (10) according to any one of claims 2 to 13, said device also comprising:
- a processing unit connected to the at least one acquisition electrode (32, 34, 33), and, if appropriate, to the at least one reference electrode in order to process information transmitted by the at least one acquisition electrode (32, 34, 33) and the at least one reference electrode;
- a unit for the transmission of the information processed by the processing unit.

15. Method for positioning an acquisition device (10) on the head of a user (12) in order to perform an encephalogram, the head of the user (12) being defined in a transverse plane (Pt), a sagittal plane (Ps) and a frontal plane (Pf), the method comprising the steps consisting of:
- providing an acquisition device (10) according to any one of claims 3 to 14;
- arranging the acquisition device (10) on the head of the user (12);
- actuating the third adjustment means (40) in order to tightly fit around the head of the user (12),
- actuating the second adjustment means (38) so as to place the upper support (30) equidistant from a distal end of the anterior transverse portion (22) and a distal end of the posterior transverse portion (24),
- actuating the first adjustment means (36) in order to adjust the position of the at least one acquisition electrode (32, 34, 33) in the frontal plane (Pf);
in which the steps of actuating the first (36, 38, 40) and second (36, 38, 40) adjustment means are performed independently.

16. Positioning method according to claim 15, also comprising the steps consisting of:
- determining a first and a second adjustment positions of the first (36, 38, 40) and second (36, 38, 40) adjustment means, respectively;
- memorizing the first and second adjustment positions;
- actuating the first (36, 38, 40) and second (36, 38, 40) adjustment means so as to position them in adjustment positions different from the first and second adjustment positions;
- removing the acquisition device (10) from the head of the user (12);
- replacing the acquisition device (10) on the head of the user (12);
- actuating the first (36, 38, 40) and second (36, 38, 40) adjustment means so as to position them according to the first and second adjustment positions.

17. Positioning method according to claim 15 or 16, in which the acquisition device (10) is an acquisition device (10) according to any one of claims 2 to 14 in combination with claims 4, 11 and 13, in which the step consisting of arranging the acquisition device (10) on the head of the user (12) comprises the steps consisting of:
- displacing the anterior support (14) with respect to the posterior support (16) so that the arched portion (11) has a circumference equal to or greater than the maximum circumference of the head of the user (12), considered in the transverse plane (Pt);
- positioning the acquisition device (10) on the head of the user (12) so that the bearing portion (46) bears on an ear of the user (12);
- positioning the acquisition device (10) on the head of the user (12) so that the hollow (48) of the posterior support (16) is facing the external occipital protuberance of the head of the user (12).
